# EUROPEAN PATENT APPLICATION

(11) **EP 3 424 597 A1**
(43) Date of publication of application: **09.01.2019**
(21) Application number: 17001149.8
(22) Date of filing: 04.07.2017
(51) Int. Cl.: B01L 3/00, C12M 1/00, C12M 3/06

(54) **METHOD FOR INFLUENCING BIOLOGICAL SYSTEMS BY PHYSICO-CHEMICAL GRADIENTS AND GENERATION THEREOF IN A MICROFLUIDIC DEVICE**

(71) Applicant: Karlsruher Institut für Technologie, 76131 Karlsruhe (DE)
(72) Inventor: Niemeyer, Christof, 28213 Bremen (DE); Rabe, Kersten, 76133 Karlsruhe (DE); Silla, Hansen, 76185 Karlsruhe (DE)
(74) Representative: Müller-Boré & Partner Patentanwälte PartG mbB

(57) **Abstract**

The present invention relates to a method for exposing at least one cell to at least one quasi-static physico-chemical gradient, a microfluidic chip, a use of a microfluidic chip for exposing at least one cell to at least one physico-chemical gradient, a kit comprising a microfluidic chip and a device comprising a microfluidic chip.

## Description

The present invention relates to a method for exposing at least one cell to at least one quasi-static physico-chemical gradient, a microfluidic chip, a use of a microfluidic chip for exposing at least one cell to at least one physico-chemical gradient, a kit comprising a microfluidic chip and a device comprising a microfluidic chip.

Cells are often used in biotechnology for production of substances, for analytical purposes or in medicinal therapy. Apart from these uses, they can also be employed for fundamental research and as model systems for investigation of metabolic processes. Specific and clearly defined chemical or physical environments are often desired for studying cellular systems. For investigating various environmental conditions simultaneously, a large number of parallel experiments is often necessary. This can be simplified by employing chemical or physical gradients.

Gradients are often found in nature and influence cellular systems, for example, gradients are present in signal transmission, differentiation of cell communities or the production of energy. In the area of cellular biotechnology, physico-chemical gradients could be employed for the following applications:
- fast determination of optimum growth conditions for an organism;
- fast determination of optimum reaction parameters for substrate turnover;
- fast separation of complex consortia of microorganisms;
- adapting of cells, organisms or consortia of organisms to altered growth conditions;
- fast identification of environmental factors which cause a specific physiological reaction (e.g. gene expression)

"Mega plates" which are agar plates with a size of 120 x 60 cm and varying antibiotics concentration across the plate can be used to visualize mutation and selection in a migrating bacteria front. However, the large size of the plates makes their production, handling and incubation difficult. Furthermore, since it is not possible to introduce additional substrate for the cells, the local environmental conditions change over time and also influence the behaviour of the cells.

Another approach for exposing cells to a chemical gradient is the use of a device which comprises two chambers with different antibiotic concentrations which are connected via a thin channel. In this way, a gradient is created between the two chambers. Cells inoculated in one chamber can migrate into the channel and once adaption has occurred can also colonize the second chamber. However, since the gradient across the channel is created via diffusion also the substrate supply relies on diffusion. Accordingly, the channel can be depleted of substrate very quickly and, additionally, due to its narrow width, it can become fully overgrown in a short time, thereby terminating the experiment.

Other approaches aim to overcome the dependence on diffusion for substrate supply by employing a microfluidic device, which exhibits microchambers with a continuous flow of growth medium. In this microfluidic device, a Christmas-tree-mixer is employed for creating a gradient of cell influencing substances. This mixer comprises eight different channels, which each contains an individual substance concentration and is each connected to an individual microchamber. Although this set-up allows it to monitor the reaction of cells to several specific constant concentrations simultaneously in a high-throughput way, it is not possible to induce adaption of the cells to different concentrations since the individual microchambers are not in contact with each other, thereby inhibiting migration of cells to different concentration regimes.

The present invention overcomes the above mentioned disadvantages. Thus, the technical problem underlying the present invention is to provide a method for exposing at least one cell to at least one physico-chemical gradient under quasi-static conditions for multiple generation times.

The solution to the above technical problem is achieved by the embodiments characterized in the claims.

In particular, the present invention provides a method for exposing at least one cell to at least one physico-chemical gradient comprising the steps of:
(a) providing a microfluidic chip comprising a cultivating chamber, wherein the microfluidic chip is suitable for generating a physico-chemical gradient in said cultivating chamber;
(b) creating a physico-chemical gradient in said cultivating chamber;
(c) inoculating the cultivating chamber with at least one cell; and
(d) incubating the at least one cell in the microfluidic chip after inoculation.

A microfluidic chip as used in step (a) according to the present invention comprises at least one inlet, at least one gradation unit, where a physico-chemical gradient is created, a cultivating chamber and at least one outlet provided downstream of the cultivating chamber, wherein the individual components are either directly connected or connected to each other via microfluidic channels.

In the following, descriptions of relative positions of microfluidic elements like "after", "into" or "out of" are to be understood in direction of the flow through the microfluidic elements, i.e. from the at least one inlet to the at least one outlet.

A microfluidic chip according to the present invention is preferably a microfluidic gradient chip and can be prepared by methods known in the art. For example, the microfluidic chip can be prepared by replica moulding of a master structure with PDMS and subsequent bonding to a glass slide to close the thus formed channels. In a preferred embodiment, the microfluidic chip has the size of a conventional microscope slide to be compatible with a variety of commercially available microscopes.

The width of the microfluidic channels perpendicular to the direction of the flow is not particularly limited. The size can be, for example, between 0.01 and 10 mm, preferably between 0.1 and 5 mm, more preferably between 0.2 and 1 mm and most preferably 0.4 mm.

The size and shape of the at least one inlet of the microfluidic chip is not particularly limited as long as it is suited for introducing fluids into the microfluidic chip. For example, it can be round with a diameter of 0.1 to 10 mm, preferably 0.5 to 5 mm, more preferably 0.75 to 2 mm and most preferably 1 mm. The at least one inlet is connected to a microfluidic channel, the so called inlet channel, which leads to the at least one gradation unit.

The at least one gradation unit of the microfluidic chip according to the present invention is not particularly limited in its shape, as long as it is suited for creating a physico-chemical gradient of the fluids introduced through the at least one inlet. According to the present invention, the gradation unit is preferably a T-mixer or a Christmas-tree-mixer.

A T-mixer comprises three channels arranged in a Y- or T-shape, wherein a T-shape is preferred. The part of the T-mixer where the three channels intersect is called the junction of the T-mixer (or T-junction). Mixing of a first and a second fluid to be mixed by using a T-mixer is performed by introducing the first fluid into a first channel and introducing the second fluid into a second channel of the T-mixer. Thereby, the first and second fluids are brought into contact at the junction of the T-mixer and continue to flow through the third channel of the T-mixer. Preferably, when the T-mixer has a T-shape, the first and second fluids are introduced into the first and second channels that oppose each other and, after the first and second fluids have come into contact, they flow through the third channel perpendicular to the first and second channels.

A Christmas-tree-mixer comprises a number of mixing units, which each consist of a distributing channel perpendicular to the flow direction and a number of parallel mixing channels in flow direction connected downstream to the distributing channel. The number of mixing channels in each mixing unit increases by 1 for every consecutive mixing unit, wherein the mixing channels may be straight or in a zig-zag shape. The first mixing unit in flow direction is connected to a number "n" of parallel and preferably equidistant inlet channels, wherein the distance between two neighbouring inlet channels is "d". The length of the first distributing channel is nxd, wherein the channels entering the distributing channel, i.e. the inlet channels for the first distributing channel and the mixing channels for all consecutive distributing channels, are arranged such that one channel each is located at 0.5 d from either end of the mixing channel and the remaining channels are distributed evenly between these two channels. The number of mixing channels leaving a distributing channel is 1 more than the number of channels entering the distributing channel. These mixing channels are arranged such that one channel is located at either end of the distributing channel and the remaining channels are distributed evenly between these two channels.

If a Christmas-tree-mixer is used for generating the physico-chemical gradient, the cultivating chamber of the microfluidic chip is located directly after the last mixing unit. In case a T-mixer is used for generating the physico-chemical gradient, an additional section for expanding the created gradient from the width of the third channel of the T-mixer to the width of the cultivating chamber is required. Such an expanding unit comprises a number of parallel channels in a certain distance from each other which are arranged perpendicular to the third channel of the T-mixer. The width of the channels perpendicular to the third channel of the T-mixer increases in a staggered way in direction of the third channel of the T-mixer, while the third channel of the T-mixer narrows towards the last channel of the expanding unit, i.e. the channel the farthest away from the T-junction, thereby enabling a homogeneous flow through the individual channels.

In a preferred embodiment, the volume of the cultivating chamber represents at least 5%, preferably at least 10%, more preferably at least 25%, even more preferably at least 50% and most preferably at least 75% of the total volume of all microfluidic components of the microfluidic chip. The volume of the cultivating chamber is at least 0.1 mm³, preferably 1 mm³, more preferably at least 5 mm³, even more preferably at least 15 mm³, most preferably at least 30 mm³, furthermore, at most 500 mm³, preferably at most 250 mm³ and most preferably at most 100 mm³.

In a preferred embodiment, the cultivating chamber has a larger volume than the volume of the channels leading to the cultivating chamber, resulting in a decrease of flow rate. In a preferred embodiment, the flow rate inside the inlet channels is from 0.01 to 100 mm/s, preferably from 0.05 to 10 mm/s, more preferably from 0.1 to 1 mm/s, even more preferably 0.3 to 0.5 mm/s and most preferably 0.38 mm/s, whereas the flow rate in the cultivating chamber is from 0.001 to 100 mm/s, preferably from 0.01 to 10 mm/s, more preferably from 0.02 to 1 mm/s, even more preferably from 0.04 to 0.06 mm/s and most preferably 0.05 mm/s. In a preferred embodiment, due to the low flow rate in the cultivating chamber, sheer forces are reduced and cells can colonize the cultivating chamber more easily without being excessively removed from the cultivating chamber by the flow of the at least one fluid. In particular, in a preferred embodiment, at least one cell is not removed from the cultivating chamber by the flow of the at least one fluid. Preferably at least 1% of the cells is not removed from the cultivating chamber by the flow of the at least one fluid, more preferably at least 10%, even more preferably at least 50%, even more preferably at least 75%, still more preferably at least 90% and most preferably at least 99% of the cells.

In another embodiment, the flow rate in the cultivating chamber is adjusted to remove cells not adhering to a surface of the cultivating chamber, while cells adhering to a surface of the cultivating chamber remain in the cultivating chamber.

In yet another embodiment, the flow rate is varied over time, in order to select for cells, which are better adhered to a surface of the cultivating chamber, by increasing the flow rate. In general the flow rates do not have to be fixed during the incubation and can be changed over time, depending on the circumstances. Thereby, it is possible to ensure that only cells adhered to a surface of the cultivating chamber stay in the cultivating chamber, thus the microfluidic chip can be used to evolve biofilms.

Preferably, the relatively large size of the cultivating chamber enables long incubation times without risking clogging of the microfluidic elements with cell aggregates. In a preferred embodiment, the cultivating chamber is additionally connected to an outlet port, which enables a constant flow of the at least one fluid through the microfluidic chip.

In a preferred embodiment, the microfluidic chip is connected to an external pump and fluid reservoir and is preferably equipped with a bubble trap to ensure a constant flow of the at least one fluid through the microfluidic chip.

In a preferred embodiment, the at least one fluid flowing through the microfluidic chip is chosen from cleaning or sterilizing fluids for subjecting a microfluidic chip to a cleaning or sterilizing step before or after exposing the at least one cell to a physico-chemical gradient.

In another embodiment, the at least one fluid flowing through the microfluidic chip is chosen from a fluid, which is a suitable growth medium for cells, for subjecting a microfluidic chip to an equilibration step before exposing at least one cell to a physico-chemical gradient and during cultivating the at least one cell during the exposure. This growth medium can be suitably chosen by a person skilled in the art according to the type of cells to be exposed to a physico-chemical gradient. The growth medium is commonly water based and has a suitable composition. According to the present invention, M9 growth medium is preferably chosen.

A physico-chemical gradient as created in step (b) and further described herein is preferably a continuous or quasi-continuous distribution of a physical and/or chemical property over a spatial region. The gradient can be generated inside the microfluidic chip or applied externally or a mixture of both. The physico-chemical gradient according to the present invention comprises at least one of a proliferation inhibitor, a proliferation amplifier or a mutagen.

The gradient generated inside the microfluidic chip can, for example, be a gradient of a concentration of one or more agents, which may have a positive, a negative or no influence on the at least one cell exposed to the physico-chemical gradient. In a preferred embodiment, the at least one agent has a positive or negative influence on the proliferation efficiency, the growth, the productivity, the stress resistance or the survival of the at least one cell exposed to the physico-chemical gradient.

Agents having a positive influence on the proliferation efficiency of the cell exposed to the physico-chemical gradient (proliferation amplifier) can be any agent required for proliferation of the cell. It can, for example, be nutrients, which the cell can process. In a preferred embodiment, the nutrient is glucose.

Agents having a negative influence on the proliferation efficiency of the cell exposed to the physico-chemical gradient (proliferation inhibitor) can be any agent interfering with the proliferation of the cell. It can, for example, be antibiotics or toxic aldehydes. In a specific embodiment, the agent is kanamycin, ciprofloxacin or furfural.

The above at least one agent is comprised in the at least one fluid introduced to the microfluidic chip through the at least one inlet. The gradient created inside the microfluidic chip by the gradation unit is a quasi-static gradient. That is the microfluidic chip is permanently supplied with fresh fluid through the at least one inlet, in a way that the concentration of the at least one agent at a certain position inside the microfluidic chip remains constant throughout the exposure of the at least one cell to a physico-chemical gradient, even if some of the initial agent is consumed by the at least one cell.

The gradient applied externally can be any gradient of a physical property generated outside the microfluidic chip, which is able to penetrate to the inside of the microfluidic chip, thereby influencing the life-cycle of the cell exposed to the physico-chemical gradient. The gradient can, for example, be differences in temperature or illumination. The illumination is preferably chosen to be an illumination with light of a wavelength interfering with the life-cycle of the cell. Preferably, the wavelength is in the ultra-violet region.

In an alternative embodiment, the illumination is chosen to be an illumination which interacts with an optical switch, which has been introduced into at least one cell to be incubated in the microfluidic chip before inoculating the microfluidic chip with the at least one cell. Methods for introducing an optical switch into a cell are known in the art and will be employed by the person skilled in the art depending on the circumstances. The optical switch is preferably related to optogenetics and will be chosen by the person skilled in the art according to the desired effect. The optical switch can, for example, be used to enable or inhibit adhesion of the at least one cell to a surface of the cultivating chamber, thereby allowing for a possible selection of cells, which carry the optical switch from cells which do not carry the optical switch by removal of the non-adhered cells with the flow through the microfluidic chip.

The physico-chemical gradient preferably extends across the width of the cultivating chamber of the microfluidic chip. Furthermore, the microfluidic chip is preferably constantly supplied with fresh fluid by the at least one inlet and discharged of old fluid through the at least one outlet so that the created gradient remains constant, even if all or parts of the gradient are disturbed by the at least one cell. Such a gradient is called quasi-static gradient.

The present invention is not limited to a particular type of cell. The at least one cell exposed to at least one physico-chemical gradient is preferably a cell derived from a cell culture or a cell derived from an individual. The cell culture preferably comprises at least one microorganism. Non-limiting examples of cells suitable for the inoculation of the cultivating chamber in step (c) are prokaryotic cells including gram-negative or gram-positive bacteria (e.g. *E*. *coli, Burkholderia spec., Bacillus spec.*) and Archaea as well as eukaryotic cells including yeast cells (such as *Saccharomyces,* e.g. *Saccharomyces cerevisiae*), insect cells (e.g. *Drosophila melanogaster*), plant cells and mammalian cells (e.g. HEK 293, HeLa). Furthermore isolates from natural or technical biofilm communities (e.g. from waste-treatment facilities, biogas producing plants, sewage plants, extreme habitats, sites of chemical spillage) or mixtures of different origin including combinations of a non-limited number of species are suitable for inoculation of and incubation in the microfluidic chip of the present invention.

The term "individual" as used herein is not limited to any particular individual and may be a fungus, an animal, a human being or a plant.

The sample taken from an individual may be any sample. In one embodiment of the present invention, the sample may be derived from a naturally occurring system, preferably a sample containing a body fluid or components derived from a body fluid. In one embodiment of the present invention, the sample may be a naturally occurring system such as a solution selected from the group consisting of serum, saliva, urine, bile, lymph, tissue, like e.g. bladder or kidney, cerebrospinal fluid and/or other body fluids in the case of an animal or human individual.

In another embodiment the sample may be derived from a naturally occurring system, preferably a sample containing a tissue or components derived from a tissue. A tissue or a component derived therefrom may be of various origins, like for example leafs, roots, petals, germs or stems in the case of plant individual, or muscle tissue, heart tissue, liver tissue, kidney tissue, epithelial tissue, connective tissue, or nervous tissue.

The term "cell culture" in its various grammatical forms, refers to cells grown in suspension, roller bottles, flasks and the like. Large scale approaches, such as bioreactors, including adherent cells growing attached to microcarriers in stirred fermentors, also are included. According to an embodiment of the present invention, cells derived from a cell culture may be derived from animals, plants or fungus. Animal cells may be derived for example from insects, fish, birds, or mammals. In a preferred embodiment, cells derived form a cell culture are mammalian cells including those of human origin, which may be primary cells derived from a tissue sample, diploid cell strains, transformed cells or established cell lines. Mammalian cells can include human and non-human cells alike. Mammalian cells of non-human origin can be monkey kidney cells, bovine kidney cells, dog kidney cells, pig kidney cells, rabbit kidney cells, mouse kidney cells, rat kidney cells, sheep kidney cells, hamster kidney cells, Chinese hamster ovarian cells or an animal cell derived from any tissue. In particular, mammalian cells can be BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, 293 cells, NS-1 cells MRC-5 cells, WI-38 cells, BHK cells, 293 cells, HeLa, MCF7 and RK-cells.

The sample may be derived from a mammal, preferably a mammal selected from the group consisting of human, mouse, rat, pig, cat, dog, horse, goat, cattle, cow, and monkey and/or others. In a preferred embodiment of the present invention, the sample is derived from a human. In another embodiment of the present invention, the sample contains isolated body fluid compounds or processed body fluids derived from a mammal, preferably a mammal selected from the group consisting of human, mouse, rat, pig, cat, dog, horse, goat, cattle, cow, and monkey and/or others. In a preferred embodiment of the present invention, the sample contains isolated body fluid compounds or processed body fluids derived from a human.

In a preferred embodiment, the sample is pre-purified. In a more preferred embodiment of the present invention, the pre-purification comprises the step of removing impurities that impair significantly or prevent the growth of the cell.

Inoculation of the cultivating chamber with at least one cell can be performed by injecting a diluted cell culture into the cultivating chamber by hydrostatic pressure. Inoculation of the cultivating chamber is preferably performed in a region of the cultivating chamber, where the conditions are suitable for cell proliferation.

The inoculation of the cultivating chamber can be performed with wild-type cells, with cells of a specially designed cell line or with cells, which have undergone an adaption by being exposed to a physico-chemical gradient before.

After inoculation of the cultivating chamber with the at least one cell, the at least one cell inside the cultivating chamber of the microfluidic chip is incubated under conditions suitable for cell proliferation for a certain time. The conditions suitable for cell proliferation can be chosen by a person skilled in the art according to the type of cell to be exposed to the physico-chemical gradient.

The duration of the incubation is not particularly limited. Preferably, the incubation is performed for a time suitable to allow adaption of the inoculated cells to the whole range of the physico-chemical gradient, thereby spreading over the whole width of the cultivating chamber. The incubation time is, for example, at least 1 hour, at least 12 hours, at least 1 day or at least 1 week. The upper limit of the incubation time is not particularly limited and will be adjusted by a person skilled in the art according to the circumstances. The upper limit of the incubation time is, for example, 1 year, 6 months or 1 month.

In a preferred embodiment, the incubation is carried out between 4°C to 100°C, preferably between 10°C and 75°C, more preferably between 17°C and 27°C, and most preferably at room temperature. In another preferred embodiment, the cells are incubated at 37°C.

The atmosphere in which the incubation is carried out is not particularly limited and can be adjusted by the person skilled in the art according to the circumstances and the type of cell to be incubated. The atmosphere may contain gases which can be processed by the at least one cell in the incubation chamber, such as CO₂, H₂, H₂S, hydrocarbons, or mixtures thereof, in addition to or instead of the naturally occurring atmosphere.

During incubation, the microfluidic chip is constantly fed with fresh liquid in order to maintain a stable physico-chemical gradient inside the cultivating chamber. The incubation is preferably performed in an incubation chamber with controlled temperature, humidity and atmosphere composition.

The progress of the adaption of the inoculated cells can be monitored by optical observation of the growth behavior of the inoculated cells. Suitable parameters for the observation are, for example, the region of the cultivating chamber in which cells grow, the size and density of the cell colonies and the rate with which the cells colonize new regions of the cultivating chamber.

Additionally, it is possible to sample the cultivating chamber of the microfluidic chip. Sampling in this context means the removal of at least one cell from predefined areas of the cultivating chamber before, during or after the incubation. These samples can be further analyzed with conventional cell analysis techniques. For example, the cell samples can be transferred to liquid or solid growth medium with a certain proliferation inhibitor and the effect of the inhibitor concentration can be monitored dependent on the region of the cultivating chamber from where the sample was taken. Other cell analysis techniques may include deep-sequencing based on DNA and RNA originated from the samples, which may be further amplified in order to detect rare genetic events, Polymerase based detection methods like PCR, or optical analysis techniques like (automated) microscopy, fluorescence in situ hybridization (FISH) and related technologies.

The present invention relates to a kit containing the microfluidic chip according to the present invention. The kit may contain e.g. fluids, pumps, tubes and/or connectors, when appropriate.

The present invention also relates to a device comprising the microfluidic chip according to the present invention, bubble traps, an incubation chamber and a tool for automated inoculation, observation and sample retrieval of the microfluidic chip.

In the device according to the present invention, the microfluidic chip is connected in such a way that the introduction of gas bubbles into the microfluidic chip is avoided. Gas bubbles can easily block the microfluidic channels, thereby disturbing or preventing the flow of the at least one fluid through the microfluidic chip and the generation of the physico-chemical gradient. The introduction of gas bubbles into the microfluidic chip can, for example, be prevented by directly or indirectly connecting the microfluidic chip to bubble traps, for example by using tubing and capillaries. The type and shape of the bubble traps is not particularly limited, as long as they serve to remove bubbles from the at least one fluid and ensure a constant and uninterrupted flow of the at least one fluid. Typically, a bubble trap comprises a container with one or more inlets and one or more outlets. The container can, for example, have a tubular shape or a tapered tubular shape and is closed except for the inlet and outlet openings. The container acts as a reservoir for the at least one fluid and is filled with the at least one fluid. The at least one inlet is preferably arranged on the side of the bubble trap, close to the top-surface. The at least one outlet of the bubble trap is preferably arranged at the top surface of the bubble trap. To ensure the output of bubble free fluid through the at least one outlet, the at least one outlet is connected to a tube inside the bubble trap, which ranges into the part of the container which is filled with the fluid and ends just above the inner bottom surface of the bubble trap (intake tube). In this way, when fluid containing bubbles is introduced through the at least one inlet, the fluid sinks down and forms the fluid reservoir of the bubble trap, while the gas forming the bubbles remains above the fluid level. By taking out the fluid near the bottom of the reservoir, where no bubbles are present, a constant and bubble free flow of the liquid is ensured through the at least one outlet. It will, of course, be acknowledged that the fluid level is not to be allowed to sink below the level, where the intake tube for the at least one outlet is positioned.

The incubation chamber of the device is suitable for containing the microfluidic chip during incubation. Furthermore, the incubation chamber allows the control and adjustment of environmental parameters, like temperature, humidity and composition of the atmosphere, inside the incubation chamber, where the microfluidic chip is contained. The incubation chamber is preferably sealed from the surrounding environment, such that the atmosphere, e.g. humidity and composition, is not influenced by the surrounding environment and parameters can be monitored and precisely adjusted.

The incubation chamber may contain heating and/or cooling elements for adjusting the temperature inside the incubation chamber. These heating and/or cooling elements may be connected to a temperature sensor and a temperature controller, which measures the temperature, compares it to a preset target temperature and regulates the heating and/or cooling elements according to the difference in the target temperature and the actual temperature inside the incubation chamber.

The heating element may be a localized heat source, which relies on diffusion for a uniform temperature distribution or it can be a spatially distributed heat source, which evenly heats the incubation chamber and/or the microfluidic chip directly. The heat source can be a resistive heating element, a thermoelectric heating element or an infrared radiative heater. The heating element is preferably a heating foil, which can be attached directly or indirectly to the microfluidic chip. When the foil is indirectly attached to the microfluidic chip, it is preferably attached to a holding element, in which the microfluidic chip sits. This holding element is preferably made of a material with a high thermal conductivity, e.g. a metal or metal alloy, like copper or brass. When the heating element is attached to a holding element, which is in thermal contact with the microfluidic chip, the microfluidic chip can easily be exchanged without the need to transfer the heating element. Suitable cooling elements include, for example, thermoelectric cooling elements, e.g. peltier elements, which can be arranged in the same way as described above for the heating element.

For control of the atmospheric composition inside the incubation chamber, the incubation chamber has one or more in- and outlets suitable for the introduction of gases. Like this, the concentration of, for example, oxygen and carbon dioxide can be adjusted to a value, which is suitable for the incubation of the microfluidic chip and this environment can be maintained for the duration of the incubation to ensure stable conditions. The humidity inside the cultivating chamber can, for example, be controlled by the introduction of humidified gases when controlling the composition of the atmosphere, or by providing a source of humidity within the incubating chamber. This source of humidity can, for example, be a reservoir of a saturated salt solution, the liquid used to cultivate the cells or water, which is in open contact with the cultivating chamber and will create an equilibrium humidity in the cultivating chamber by evaporation. A control of the humidity of the atmosphere surrounding the microfluidic chip can, for example, prevent the microfluidic chip from drying out.

The incubation chamber further comprises through-holes, which can be used to connect the microfluidic chip to the outside of the cultivating chamber, e.g. to a pump, thereby allowing for the introduction of the at least one fluid into the microfluidic chip, or they can be blocked when they are not in use.

Moreover, the device comprises a tool for automated inoculation, observation and/or sampling of the microfluidic chip. This tool preferably comprises a camera, a movable robotic arm, a robotic deck, and a pumping unit.

The camera of the tool can, for example, be used to recognize the position of the robotic deck or elements positioned on the robotic deck either in absolute coordinates or in relation to the robotic arm. This can be achieved by optical markers positioned on the robotic deck or on the individual elements positioned on the robotic deck. The camera can also be used for visual observation of the microfluidic chip during incubation of the at least one cell in the cultivating chamber.

The robotic arm is mounted above the robotic deck and is capable of performing precise movements in X-Y-Z-direction. This can be achieved by attaching the robotic arm to linear stages driven by step motors. Precise in this context means that the robotic arm can move with a precision of 25 µm or less in x- and 10 µm or less in y- and z-direction. The robotic arm may further comprise a means for sampling and inoculating the microfluidic chip. This means may, for example, be at least one cannula attached to the robotic arm and connected to one or more automated syringes or syringe pumps used for taking in and ejecting liquids through the at least one cannula. For controlled inoculation and sampling of the microfluidic chip, the means for sampling and inoculating the microfluidic chip may be attached to the robotic arm via a force-measuring device. This force measuring device may be a load cell, capable of detecting and measuring a force in a certain direction. This force measuring device is used in the present tool for, for example, detecting a penetration of the cannula to the inside of the cultivating chamber through the upper surface of the microfluidic chip or for detecting the bottom of a container, for example a multiwell plate arranged on the robotic deck.

In a preferred embodiment, the cannula attached to the robotic arm is connected to two or more automated syringes or syringe pumps, wherein at least one automated syringe or syringe pump is capable of manipulating small amounts of liquids and at least one automated syringe or syringe pump is capable of manipulating larger amounts of liquid. A manipulation of small amounts of liquid is, for example, necessary when inoculating or sampling the cultivating chamber of the microfluidic chip without disturbing the flow of the at least one fluid through the cultivating chamber and the physico-chemical gradient. Small amounts of liquids in this respect are for example 10 µL or less, 5 µL or less or 2.5 µL or less. A manipulation of larger amounts of liquid is, for example, necessary for flushing the system comprising the cannula and the respective connecting tubes with, for example, a cleaning fluid, a disinfecting fluid or a fluid which is also present in the microfluidic chip. Larger amounts of liquid in this respect are, for example, 50 µL or more, 100 µL or more, 500 µL or more or 1 mL or more.

The robotic deck is positioned underneath the robotic arm and preferably comprises an arrangement of various elements in a variable manner. These elements can, for example, be chip holders, holders for commercially available multiwell plates, reaction tube holders or washing stations. The chip holders are shaped in a way that they can accommodate one or more microfluidic chips. Further, the chip holder may comprise a lid, sealing the microfluidic chip inside the chip holder from the outside. The lid may further comprise predefined access points for the inoculation or sampling of the microfluidic chip by the robotic arm. In a preferred embodiment, the chip holder is temperature controlled and comprises a heating element and a temperature sensor, connected to a temperature controller, which controls the temperature of the chip holder. When the chip holder is temperature controlled, the chip holder preferably further comprises at least one preheating loop, wherein the at least one fluid to be introduced to a microfluidic chip inside the chip holder is guided through a part of the chip holder, which is temperature controlled in order to adjust the temperature of the at least one fluid to be introduced to the microfluidic chip to the temperature inside the chip holder. The chip holder may further comprise one or more compartments for accommodating one or more bubble traps, which are preferably connected in between the preheating loop and the microfluidic chip.

In a preferred embodiment, the chip holders act as the incubation chamber and possess all the features of the cultivating chamber described above. In a different embodiment, the robotic deck and/or the tool for automated inoculation, observation and/or sampling is placed inside the cultivating chamber.

As described above, the robotic deck may further comprise holders for commercially available multiwell plates. These plates can, for example, be used to individually collect the samples taken by the tool for automatic sampling. In the same way, the holders for reaction tubes can be used to provide reaction tubes where the samples taken from the microfluidic chip can be subjected to certain reactions. These reactions can be, for example, analytical reactions for analyzing the samples taken from the microfluidic chip or they can be used for preparing the retrieved samples for further use. The multiwell plates may also be used to provide liquids for the inoculation of the microfluidic chip.

The washing stations, which may be arranged on the robotic plate, are used to rinse and flush the cannula and corresponding tubing attached to the robotic arm before and/or after the inoculation or sampling of the microfluidic chip. The washing stations may comprise flushing wells for rinsing and flushing the cannula and one or more discarding wells for discarding contaminated fluids. The discarding well may comprise an outlet to remove the contaminated fluids from the discarding well so as to prevent overflowing of the discarding well.

In a typical sampling step, the previously washed cannula is moved over the cultivating chamber of the microfluidic chip and moved down to penetrate through the surface of the microfluidic chip into the cultivating chamber. Then, a sample is taken by operating the automated syringe or syringe pump and the cannula is retracted from the microfluidic chip. The sample is then dispensed in one well of a multiwell plate or in a reaction tube by moving the cannula above the respective container and operating the automated syringe or syringe pump in the opposite way as before, followed by washing of the cannula in the washing station.

The device according to the present invention may be operated in an automated fashion and controlled by a control unit. The control unit is thereby capable of activating the individual functions of, for example, the robotic arm, the automated syringes or syringe pumps and the camera, and of receiving information from, for example, a temperature sensor, a humidity sensor or the camera, and of using the obtained information in order to operate the device in a predefined manner. In a preferred embodiment, the control unit is capable of monitoring and maintaining and/or adjusting certain environmental parameters, such as temperature, humidity or atmospheric concentration in the incubation chamber or the flow of at least one fluid through the microfluidic chip. In a further preferred embodiment, it is possible to define a routine according to which the control unit operates the device. This can for example be an automated sampling routine, which samples the microfluidic chip at predefined positions at predefined times and performs the necessary washing steps.

In a preferred embodiment, it is possible to operate and program the control unit via a graphical user interface.

When the device comprises the above described elements, it is suitable for long-term incubation of the microfluidic chip of the present invention without the risk of unwanted distortion of the incubation conditions by, for example, gas bubbles or changes in temperature, humidity or composition of the atmosphere.

In summary, the present invention offers the following advantages:
- The size of the cultivating chamber and the constant supply of fresh fluid allows incubation times of multiple generations, thereby increasing the probability of adaption of the at least one cell to different environmental regimes within the cultivating chamber;
- The different environmental regimes are not self-contained but in direct contact with each other and can thus be colonized by cell migration, starting from the inoculation point;
- The progress of the adaption of cells to varying physico-chemical environments can be monitored in real-time by visual observation or intermediate sampling.
- The conditions inside the cultivating chamber can be adjusted during the incubation in order to promote adaption or selection for specific cells.
- The microfluidic chip can be prepared from cheap materials and is easy to use.

The Figures show:
- Fig. 1:: A) Possible design for generating a gradient of two different fluids with a T-mixer. Measurements in mm. Left: Topview; right: crossection A.
B) Possible alternative design for generating a gradient of two different fluids with a Christmas-tree-mixer. Left: Topview; right: crossection A.

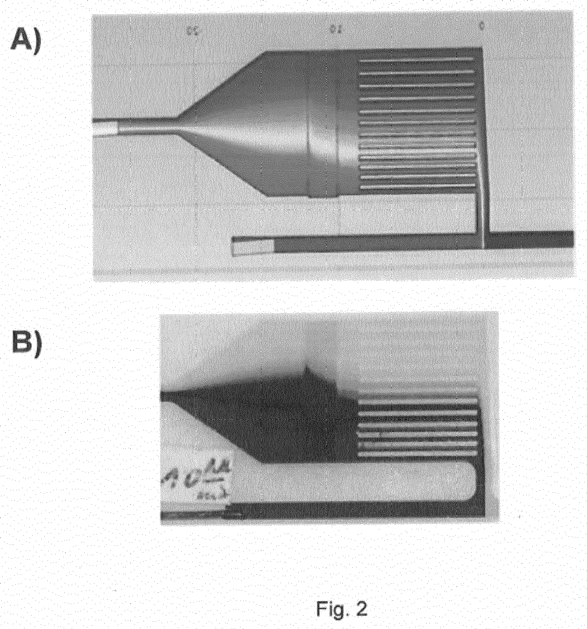

- Fig. 2:: Possible design for generating a gradient of two different fluids with a T-mixer.
Top: theoretical gradient calculated by numerical simulation (comsol). Fluid 1: red; fluid 2: blue.
Bottom: channels formed in PDMS in which fluid 1 (coloured blue with xylene cyanol) and fluid 2 (coloured yellow with orange G) mix according to the simulation at a flowrate of 10 µL/min.

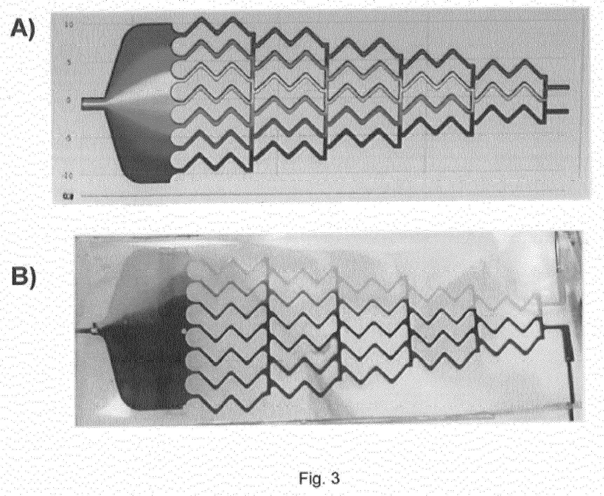

- Fig. 3:: Possible design for generating a gradient of two different fluids with a Christmas-tree-mixer.
Top: theoretical gradient calculated by comsol multiphysics modelling software fluid 1: red, fluid 2: blue.
Bottom: channels formed in PDMS in which fluid 1 (coloured blue with xylene cyanol) and fluid 2 (coloured yellow with orange G) mix according to the simulation at a flowrate of 10 µL/min.
- Fig. 4:: Use of a gradient of two different fluids with a T-mixer for adaption of a bacterial isolate to proliferation under high antibiotics concentrations (here: kanamycin).
A) Theoretical gradient calculated by numerical simulation (comsol). Fluid 1: red (defined M9 growth medium with 2.2 mM glucose and 100 mg/L kanamycin); fluid 2: blue (defined M9 growth medium with 0 mM glucose and 0 mg/L kanamycin). The microfluidic chip has been inoculated with cells sensitive to kanamycin at position 1. After a defined cultivation time of 7 days under constant conditions (5 µL/min, 28°C), a sample of the adapted bacteria was taken from position 2.
B) Liquid growth medium (M9) supplemented with increasing glucose and kanamycin concentrations was inoculated with adapted cells from a sampling position with a high kanamycin concentration (∼100 mg/L, black circles) and with cells from a second sampling position with a medium kanamycin concentration (∼50 mg/L, grey squares) and incubated for 36 h at 28°C. Subsequently, the optical density was measured. Only adapted cells were able to proliferate at higher antibiotics concentrations.
C) A second adaption in a microfluidic chip as described in A) has been performed with the bacterial isolate, which was adapted earlier to a kanamycin concentration of 100 mg/L. Fluid 1: red (defined M9 growth medium with 2.2 mM glucose and 200 mg/L kanamycin); fluid 2: blue (defined M9 growth medium with 0 mM glucose and 100 mg/L kanamycin). Liquid growth medium (M9) supplemented with increasing glucose and kanamycin concentrations was inoculated with adapted cells from a sampling position with a high kanamycin concentration (-200 mg/L, black circles) and with cells from a second sampling position with a medium kanamycin concentration (∼150 mg/L, grey squares) and incubated for 36 h at 28°C. Subsequently, the optical density was measured. Only adapted cells were able to proliferate at higher antibiotics concentrations.

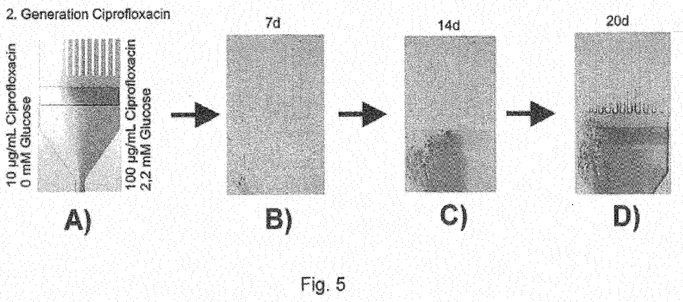

- Fig. 5:: Adaption of a bacterial isolate to proliferation under high antibiotics concentration (here: ciprofloxacin).
Left: gradient at a flow rate of 5 µL/min, visualized by coloured fluids. Fluid 1: white (defined M9 growth medium with 0 mM glucose and 0 mg/L ciprofloxacin), fluid 2: grey (defined M9 growth medium with 2.2 mM glucose and 100 mg/L ciprofloxacin).
From left to right: photographic images after cultivating times of 7 (B), 14 (C) and 20 days (D) under constant conditions (5 µL/min, 28°C). Cells growing at the right hand edge of the cultivating chamber after 20 days (image D) are adapted to high antibiotics concentrations present in this region of the cultivating chamber and can thus grow.

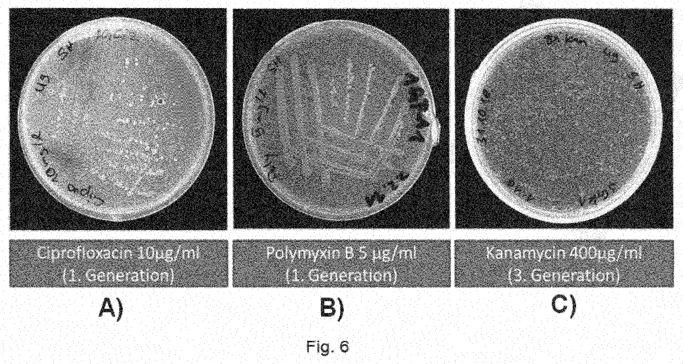

- Fig. 6:: Cells from a bacterial isolate as individual cultures on solid substrate (agar plate with M9 growth medium, with glucose and various antibiotics) after adaption to different, for the original culture inhibiting antibiotics concentration. All adapted cultures show proliferation under these initially inhibiting conditions and homogeneous morphology.

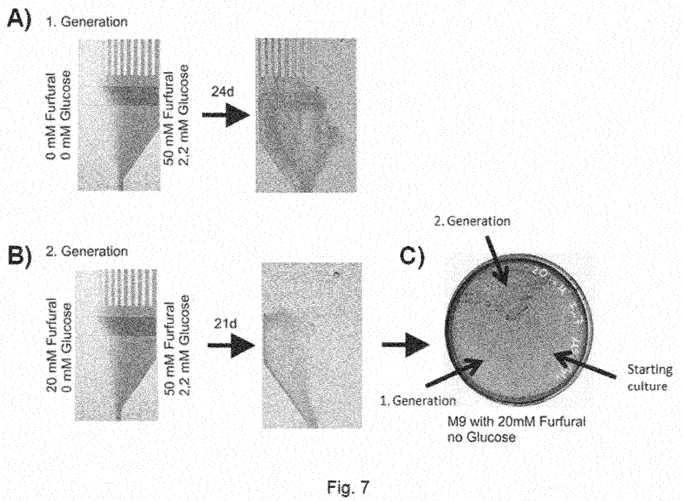

- Fig. 7:: Adaption of a bacterial isolate to growth on a toxic substrate (here: furfural). First, adaption to high furfural concentrations in growth medium (A), then growth on furfural as single carbon source (B).
A) left: gradient at a flow rate of 5 µL/min, visualized by coloured fluids. Fluid 1: white (defined M9 growth medium with 0 mM glucose and 0 mM furfural); fluid 2: grey (defined M9 growth medium with 2.2 mM glucose and 50 mM furfural).
   right: photographic images after 24 days of cultivation under constant conditions (5 µL/min, 28°C). Growth at high furfural concentrations is possible.
B) left: gradient at a flow rate of 5 µL/min, visualized by coloured fluids. Fluid 1: white (defined M9 growth medium with 0 mM glucose and 20 mM furfural); fluid 2: grey (defined M9 growth medium with 2.2 mM glucose and 50 mM furfural).
   middle: photographic images after 21 days of cultivation under constant conditions (5 µL/min, 28°C). Even with no glucose present, growth is possible at moderate furfural concentrations.
   right: Growth of the bacterial isolate on solid substrate (agar plate) with 20 mM furfural as only carbon source in M9 growth medium.
   The adapted cultures show proliferation even under furfural concentrations lethal for the original culture and show homogeneous growth. After the second adaption in a microfluidic chip, the cells are adapted to not only tolerate furfural, but also to use furfural as only carbon source in M9 minimal growth medium.

The present invention will be further described with the following non-limiting examples.

### Example 1: Fabrication of a microfluidic chip

A microfluidic chip comprising a T-mixer for creating a physico-chemical gradient, a cultivating chamber and an expanding unit for expanding the physico-chemical gradient created in the T-mixer to the width of the cultivating chamber has been fabricated as follows.

A master structure for replica-moulding of a microfluidic chip has been prepared from a brass block by CNC-milling (computerized numerical control). Cannulas (Sterican, B. Braun Melsungen AG, Germany), which were fixed to the master structure by horizontal drillholes, were used as spacers for the final connectors during moulding with PDMS pre-polymer (Sylgard 184, Dow Corning, USA). The PDMS was hardened for 3 hours at 60 °C before being removed from the mould. The PDMS was subsequently activated by being exposed to an oxygen plasma (2 minutes, 20 sccm, 300 W, Plasma Flecto 10, Plasma technology, Germany) and covalently bonded to a glass cover slip.

### Example 2: Creating a physico-chemical gradient

A microfluidic chip of Example 1 was connected to a multichannel syringe pump (KDS Multi syringe infusion/withdrawal pump series 230, KD Scientific, USA) using silicone tubes and sterile cannulas. All components were sterilized before use and assembled under sterile conditions.

The whole system was flushed with isopropanol (70 vol%) and all air bubbles were removed. Afterwards, the isopropanol was replaced with M9 minimal growth medium (33.7 mM Na₂HPO₄, 22 mM KHPO₄, 8.55 mM NaCl, 9.35 mM NH₄Cl, 1 mM MgSO₄, 0.3 mM CaCl₂, biotin, thiamine, trace elements, glucose) at a flow rate of 5 µL/min at each inlet. The whole system was left to equilibrate over night at a flow rate of 5 µL/min.

To create the physico-chemical gradient in the cultivating chamber of the microfluidic chip of Example 1, two different fluids are introduced to the microfluidic chip through the inlets at equal flow rates and brought into contact in the T-Mixer without being convectively mixed. A gradient of the two fluids is created across the width of the third channel of the T-mixer by diffusion, which is expanded to the size of the cultivating chamber by the expanding unit.

### Example 3: Adaption of a bacterial isolate to kanamycin

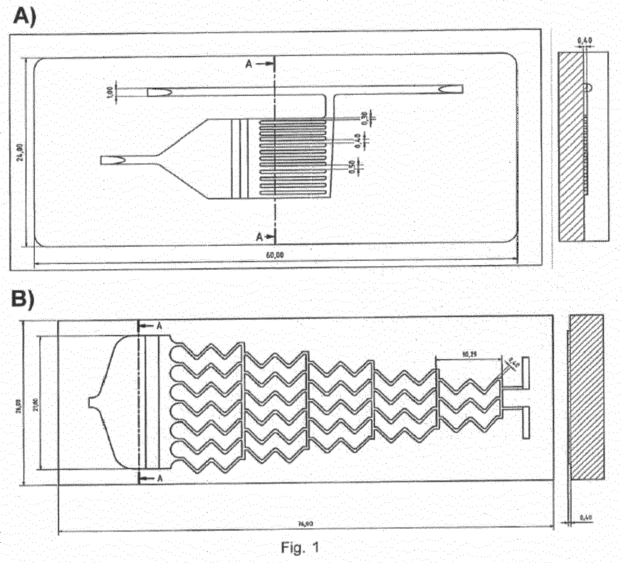

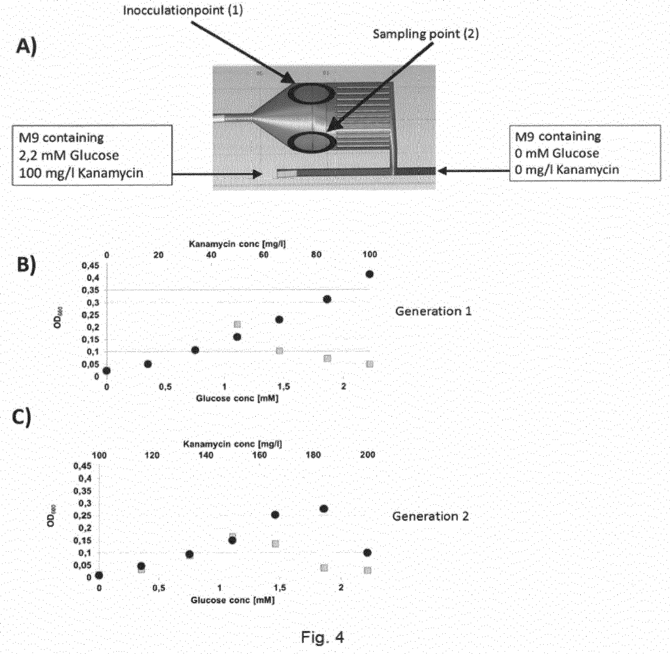

The microfluidic chip of Example 1, depicted in Figure 1A), was used for a three-step adaption of a bacterial isolate to the glycosidic antibiotic kanamycin. For the first step, a kanamycin gradient, which ranged from 0 mg/L to 100 mg/L, was created parallel to a glucose gradient ranging from 0 mM to 2.2 mM (Figure 4A). Adapted cells were collected from a region with a medium kanamycin concentration (50 mg/L) and from a region with a high kanamycin concentration (100 mg/L, first generation cells). These cells were used to inoculate a concentration series of kanamycin in a liquid growth medium. Only cells adapted to a high kanamycin concentration showed proliferation after 36 hours at a kanamycin concentration of 100 mg/L (Figure 4B).

In a second step, the kanamycin gradient in the microfluidic chip was changed to range from 100 mg/L to 200 mg/L parallel to a glucose gradient ranging from 0 mM to 2.2 mM and inoculated with adapted cells of the first generation.

After five days samples were collected from a region with a medium kanamycin concentration (150 mg/L) and from a region with a high kanamycin concentration (200 mg/L, second generation cells). These cells were used to inoculate a concentration series of kanamycin in a liquid growth medium. Only cells adapted to a high kanamycin concentration showed proliferation after 36 hours at a kanamycin concentration higher than 150 mg/L (Figure 4C).

In a third step, the kanamycin gradient in the microfluidic chip was changed to range from 200 mg/L to 400 mg/L parallel to a glucose gradient ranging from 0 mM to 2.2 mM and inoculated with adapted cells of the second generation. This kanamycin concentration corresponds to the 200-fold minimum inhibitory concentration of the initial bacterial isolate. After adaption of the cells to the high kanamycin concentration, samples were collected (third generation cells), which were able to proliferate on an agar plate with a kanamycin concentration of 400 mg/L (Fig. 6C)

### Example 4: Adaption of a bacterial isolate to ciprofloxacin

The microfluidic chip of Example 1, depicted in Figure 1A), was used for a three-step adaption of a bacterial isolate to the gyrase inhibitor ciprofloxacin. In the same manner as for the adaption to kanamycin (Example 3), a glucose gradient (0 mM to 2.2 mM) was created parallel to the antibiotics gradient. In the first step, the cells were exposed to a ciprofloxacin gradient ranging from 0 mg/L to 10 mg/L. After adaption of the cells to a ciprofloxacin concentration of 10 mg/L (10 days), samples were collected (first generation cells). These cells showed proliferation in liquid growth medium as well as on solid agar plates (Fig. 6A) at this antibiotics concentration, which corresponds to approximately the 300-fold minimum inhibitory concentration of the initial bacterial isolate.

Figure 5 shows sequential photographic images of the second step of the adaption to ciprofloxacin with a ciprofloxacin gradient ranging from 10 mg/L to 100 mg/L taken after 7, 14 and 20 days. After 7 days initial colonization is clearly visible in regions with a lower antibiotics concentration, after 14 days regions with a ciprofloxacin concentration up to 50 mg/mL are occupied and after 20 days the cells are fully adapted to ciprofloxacin concentrations of 100 mg/L (second generation cells).

In the third step of the adaption, the maximum ciprofloxacin concentration was increased to 1000 mg/L and the second generation cells were subsequently successfully adapted to this high concentration.

In summary, in just three steps, it was possible to adapt the initial bacterial isolate with increasing quasi static ciprofloxacin concentration gradients to a concentration corresponding to the 30000-fold initial inhibition concentration.

### Example 5: Adaption of a bacterial isolate to furfural

In contrast to common antibiotics which generally target a specific functional molecule, toxic aldehydes, like furfural, can interfere with many different processes in a cell. An adaption to such a cell poison is thus very complex.

The microfluidic chip of Example 1, depicted in Figure 1A), was used for adaption of a bacterial isolate to the aromatic aldehyde furfural. A furfural concentration gradient ranging from 0 mM to 50 mM was created parallel to a glucose gradient ranging from 0 mM to 2.2 mM inside the microfluidic chip and inoculated with a bacterial isolate. Figure 7A shows a photographic image of the populated cultivating chamber after incubation for 24 days at 28°C. Cells taken as a sample (first generation cells) already showed a tolerance increased from 20 mM to 35 mM towards furfural.

In a subsequent adaption experiment, a furfural concentration gradient ranging from 20 mM to 50 mM furfural was inoculated with first generation cells, wherein the growth medium with a lower furfural concentration was again not supplemented with glucose and the growth medium with higher furfural concentration was supplemented with 2.2 mM glucose. Figure 7B shows a photographic image of the populated cultivating chamber after 21 days of incubation. Isolated cells (second generation) showed proliferation in liquid culture as well as on solid agar plates with M9 growth medium with 20 mM furfural as only carbon source, while the initial a bacterial isolate was not able to grow under these conditions.

Figure 7C shows a comparison of the growth of the initial bacterial isolate, first generation cells and second generation cells on solid M9 substrate with furfural as carbon source.

## Claims

1. A method for exposing at least one cell to at least one physico-chemical gradient comprising the steps of:
(a) providing a microfluidic chip comprising a cultivating chamber, wherein the microfluidic chip is suitable for generating a physico-chemical gradient in said cultivating chamber;
(b) creating a physico-chemical gradient in said cultivating chamber;
(c) inoculating the cultivating chamber with at least one cell; and
(d) incubating the at least one cell in the microfluidic chip after inoculation.

2. The method according to claim 1, wherein the method contains the step of
(a') creating a physico-chemical gradient with a T-mixer or a Christmas-tree-mixer, wherein
the step (a') is performed before the step (b).

3. The method according to claim 1 or 2, wherein the at least one physico-chemical gradient includes a gradient of a proliferation inhibitor and/or a gradient of a proliferation amplifier.

4. The method according to any one of claims 1 to 3, wherein the at least one cell is a cell culture.

5. The method according to any one of claims 1 to 4, wherein the cell culture comprises at least one microorganism.

6. The method according to any one of claims 1 to 5, wherein the microorganism is selected from prokaryotic cells, eukaryotic cells or mixtures of multiple microorganisms.

7. The method according to any one of claims 1 to 6, wherein the cultivation chamber is permanently fed with fresh growth medium during incubation.

8. The method according to any one of claims 1 to 7, wherein the method further comprises the steps of
(e) harvesting at least one cell from a predetermined area of the cultivating chamber after incubating; and
(f) using the at least one cell harvested in step (e) for inoculating a cultivating chamber of a microfluidic chip according to step (c).

9. The method according to any one of claims 1 to 8, wherein the biological system is evaluated after incubation, wherein the evaluation comprises the steps of:
- harvesting at least one cell from a predetermined area of the cultivating chamber; and
- subjecting the harvested at least one cell to a method of analysis.

10. The method according to any one of claims 1 to 9, wherein the microfluidic chip comprises:
- at least one inlet;
- at least one gradation unit located downstream of the at least one inlet;
- a cultivating chamber directly or indirectly connected to the outlet of the at least one gradation unit; and
- at least one outlet provided downstream of the cultivating chamber.

11. A microfluidic chip comprising a cultivating chamber containing at least one cell, wherein at least one physico-chemical gradient is created inside the cultivating chamber.

12. Use of a microfluidic chip for exposing at least one cell to at least one physico-chemical gradient.

13. The use of a microfluidic chip, wherein the microfluidic chip is the microfluidic chip according to claim 11.

14. A kit comprising the microfluidic chip according to claim 11.

15. A device comprising the microfluidic chip according to claim 11, one or more bubble traps, an incubation chamber and a tool for automated inoculation, observation and sample retrieval of the microfluidic chip.
